# EUROPEAN PATENT APPLICATION

(11) **EP 4 497 378 A1**
(43) Date of publication of application: **29.01.2025**
(21) Application number: 23187596.4
(22) Date of filing: 25.07.2023
(51) Int. Cl.: A61B 5/097, A61B 5/08, G01N 21/3504, G01N 33/497, G01N 33/00

(54) **BREATHALIZER DEVICE, METHOD AND USE**

(71) Applicant: Nederlandse Organisatie voor toegepast-natuurwetenschappelijk Onderzoek TNO, 2595 DA 's-Gravenhage (NL)
(72) Inventor: DURÁN GIMENEZ, Dinesh, 2595 DA 's-Gravenhage (NL)
(74) Representative: V.O.

(57) **Abstract**

The present disclosure relates to a breathalyzer device (1) for detecting presence of a gaseous component, such as nitrous oxide in exhaled breath. The device comprises an inlet (2), a measuring volume (4) fluidly connected between the inlet and an outlet (3), and an IR spectrophotometer assembly (10) operably configured to probe contents of the measuring volume at least at a first probing frequency in correspondence with a first adsorption band of said component, such as N₂O.

The present disclosure further relates to a method of detecting nitrous oxide (N₂O) in the breath of a user and the use of the device or method to detect whether the person partook in a prior nitrous oxide consumption or for public safety control, for example traffic safety control.

## Description

### TECHNICAL FIELD AND BACKGROUND

The present disclosure relates to a breathalyzer device. Specifically to a device for determining a level of nitrous oxide in undiluted exhaled breath of a subject. The present disclosure relates further relates to detection of nitrous oxide in the breath of a user, and to a use of the nitrous oxide detection for drug and safety control.

Nitrous oxide is used in numerous commercial and/or professional applications. Uses include application as a blowing agent and as anesthetic in health care. Recently nitrous oxide has increasingly attracted attention in in view of public recreational use as a drug.

Recreational use, inhalation, of nitrous oxide in private and/or public space increasingly raises personal and societal safety concerns. For example, recreational use of nitrous oxide is known to adversely affect a person's ability to safety partake in work, traffic or other complex operations including handling of (heavy machinery), even long after an intake. Additionally, recreational intake of nitrous oxide is known to relate adverse health conditions, for example nervous degeneration, especially after prolonged use.

Conventional breathalyzers, e.g. as used by police/enforcement services, to detect an alcohol level in exhaled breath are incapable of detecting nitrous oxide.

Hatech markets a pumped portable N₂O analyser (G200) that is configured to monitor presence/leakage of N₂O within the built environment (e.g. operation, laboratory or other rooms where N₂O is used professionally or stored.).

EP2444791A1 relates to a gas analyzer for measuring at least two components of a gas. The device is configured to monitor N₂O levels in anesthetic applications. Specifically, to determine nitrous oxide at anesthetic levels for respiratory gas/air feed for an intubated patient.

Accordingly, there remains a need for devices and/or methods that can directly detect undiluted nitrous oxide in exhaled breath.

### SUMMARY

The invention is as disclosed herein below is based on a realization by the authors, that contra to common perception, that nitrous oxide (laughing gas) can be detected in the exhaled breath of a subject long after inhalation thereof. Specifically inventors identified that N₂O, despite being a volatile gaseous compound, can be identified in exhaled breath for many hours after an intake.

The realization is implemented in a device/method configured to provide a way of positively identifying whether a person partook in a prior (recreational) use of nitrous oxide.

The device, also referred to as breathalyzer device is configured for determining a level of nitrous oxide (N₂O) in exhaled breath. As will be clear for the description herein below the device/method can be configured to detect a presence and or level of different gaseous/volatiles in exhaled breath or even a combination of comments. Advantageously, the device as disclosed herein can be a portable handheld device.

The device comprises at least an inlet for receiving a gaseous test flow, specifically exhaled breath from a subject (person to be tested), an outlet for releasing the gas flow, a measuring volume that is fluidly connected between the inlet and an outlet, and an infrared (IR) spectrophotometer assembly. The spectrophotometer is operably configured to probe contents of the measuring volume at least at a first probing frequency in correspondence with a first adsorption band the compound of which a presence is to be detected, e.g. of N₂O. For N₂O Suitable bands include bands centered around about 2200-2250 cm⁻¹, 3400-3450 cm⁻¹, 2550-2600 cm⁻¹. Alternatively, or in addition, one or more of the bands in the so-called finger print region (500-1500 cm⁻¹) can be used, e.g. at about 1250-1300 cm⁻¹ and at about 600cm⁻¹. For other compounds, probing more of more a different bands can be probed as needed . For example, ~2100-2200 cm⁻¹ in case of carbon monoxide, ~2300-2400 cm⁻¹ for CO₂, ∼2900 - 3000 cm⁻¹ for ethanol and ~2700-2900 cm⁻¹ for detection of acetaldehyde.

Accordingly, presence of the target compound, e.g. nitrous oxide, in the probed volume can be confirmed by measuring an absorbance / transmittance at a corresponding wavelength. A concentration can be determined from recorded absorbance / transmittance values.

To allow selection of one or more target probing bands the device can be fitted with one or more selectable bandpass filter disposed in a pathway between a light source and detector of the spectrophotometer assembly. For example, a rotatable, slidable or otherwise adjustable filter having a plurality of marked positions each with a filter for a specific band. In this way one device can be used to detect presence e of a plurality of compounds, e.g. compounds relevant to first responders (e.g. medical, police, and/or fire department staff).

To remove interference of with certain compounds, e.g. carbon dioxide a trap can be provided upstream the measurement volume. Preferably, the trap is provided as a removable module. Preferably, the trap comprises a specific chemical adsorbent such as a soda lime granulate to trap CO₂. In an advantageous configuration the trap is a user replaceable module, e.g. between subsequent measurement routines. Advantageously, the device, with CO2 trap, can directly analyze breath as exhaled by a user for a presence of N₂O. In use the person can advantageously blow directly into the device (e.g. via mouth and/or nose, typically mouth). The device can directly analyze the as exhaled gas flow while relying on user sustained flow.

In contrast equipment directed for room monitoring (e.g. operation room or recovery room), or equipment monitoring a respiration air feed, typically requires external flow generation means (e.g. pumps) and/or is generally subjected to much lower CO₂ levels (about 400 ppm versus about 4% for exhaled breath).

The inlet typically comprises a mouth piece, for hygienic reasons preferably a removable mouth piece, e.g. a sleeve, shaped so that a subject can comfortably blow into the device. Since the flow in the device is sustained by a user blowing into the device no pumps for forced airflow are required.

In a strongly preferred variation the analyzer is further configured to probe at a second probing frequency overlapping a second adsorption band of the target compound (e.g. N₂O), different from the first. Inventors found that by probing at multiple frequencies can mitigate or eliminate so-called false positives, e.g. due coincidental overlap of one of the probed sorption bands with a band of other volatiles/gaseous substances present in the probed sample including but not limited to: volatiles from food/beverages, (cigarette) smoke, perfumes, etc. In a variation a positive reading as to the presence of target gas can be determined by monitoring a ratio of the spectroscopic responses at the first and the second probing frequencies, e.g. a response at a first band 2200-2250 cm⁻¹ and a second band at around 3400-3450 cm⁻¹.

Preferably, the breathalyzer further comprising a flow meter. The flow meter can be used to determine / confirm whether the user provides a minimum pre-scribed flow rate over the course of a measurement routine and/or to (in combination with a timer) determine a sampled volume.

In another or further preferred embodiment, the breathalyzer further comprising an flow restriction means, e.g. an orifice, configured to restrict an inward flow rate towards the measuring channel.

Alternatively, or in addition, the breathalyzer further comprises a pressure sensor. The pressure sensor can advantageously be configured to, upon reaching a pre-determined pressure threshold, trigger a measurement period for the IR analyzer and/or the flow sensor.

In some embodiments, the breathalyzer further comprising a sorption module connected downstream the measuring volume and configured for collecting target compounds, such as nitrous oxide, from the sampled gas flow. Preferably, the sorption module is a reversibly mountable module. The sorption module can for example comprise a physisorption based sorbent such as a molecular sieves sorbent. In line capturing of compounds from the sampled exhaled gas flow can advantageously be used to confirm and/or further quantify a reading e.g. a N₂O reading. For example, using a secondary measurement method based on a different operating principle. For example, in a preferred measurement routine the device is provided with an N₂O-sorption module (e.g. a Tenax sorbent tube having a 5 Angstrom molecular sieve). The quantity of exhaled N₂O retained in the tube can subsequently be determined externally, e.g. in using thermal desorption in combination with gas chromatography-mass spectrometry (GC-MS). The external verification of target gas (e.g. N₂O) from the sample sampling volume serves as an additional fallback against potential false positives.

In a preferred embodiment, the device further comprising an output module for communicating an output reading of the device, e.g. a display and/or an audible alarm.

The present disclosure further provides a method of detecting target gas /volatile in the breath of a user. For detection of N₂O the method comprises at least the steps of: providing a breathalyzer, preferably the device according to the present disclosure, comprising at least a measuring volume fluidly connected between an inlet and an outlet of the breathalyzer, and an IR analyzer operably configured to probe contents of the measuring volume; exhaling into the inlet thereby providing a flow of exhaled gas flow through the measuring channel, and obtaining a response of the IR analyzer at a first probing frequency in correspondence with a first adsorption band of N₂O.

In a preferred variation the method further comprises obtaining a response of the IR analyzer at a second probing frequency in correspondence with a second adsorption band (e.g. of N₂O) and determining a ratio between the response at the first probing frequency and at the second probing frequency.

As described in relation to the device the method can further comprising determining a flow rate or flow volume. Among others the volume can be used to, in combination with a quantity of N₂O as determined, e.g. from the N₂O-sorption module, derive a concentration of laughing gas the exhaled breath.

In another or further preferred embodiment, the method comprises passing the exhaled gas over an N₂O-sorbent (downstream the measuring volume) thereby collecting nitrous oxide from the probed sample volume, followed by determining an amount of collected nitrous oxide. Determining the amount typically involves releasing nitrous oxide from the sorbent and measuring the amount of released nitrous oxide, e.g. by GC-MS.

In other or further embodiments, the method comprises, determining whether a volume or pressure of breath exhaled into the inlet exceeds a minimum threshold value.

The invention further provides: a use of analyzing exhaled breath by a person for a presence of nitrous oxide to determine whether the person partook in a prior nitrous oxide consumption; and a use of the method or the device according as disclosed, for public safety control, for example traffic safety control.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1 schematically illustrates a breathalyzer;
FIG 2 schematically illustrates a breathalyzer; and
FIG 3 provides experimental details.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

As used herein a the term portable handheld device refers to devices having a dimensioning and mass suitable for human handling/operation. Typically, the handheld device includes a power supply, battery, configured to operate the device over a relevant period.

In the description herein below features relating to the device, method, and or the use thereof will be exemplified with reference to the detection of N₂O. It will be appreciated that the device/method can be also be used to detect other compounds unless clear from context.

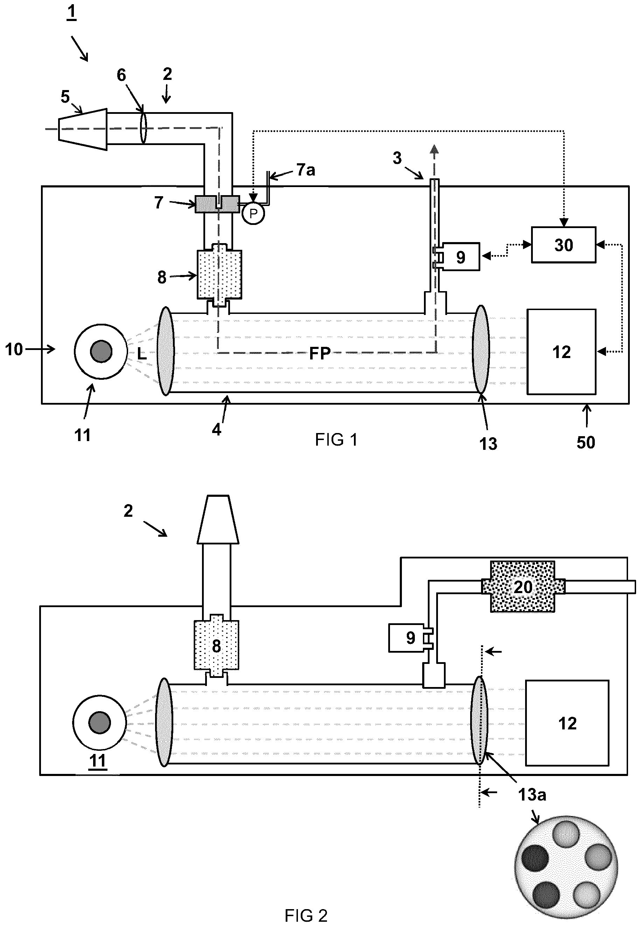

FIG 1 schematically illustrates an embodiment of the breathalyzer device 1 as disclosed herein.

The breathalyzer device comprises a housing 50 having at least an inlet 2, a measuring volume 4 fluidly connected between the inlet and an outlet 3, and an IR spectrophotometer assembly 10 operably configured to probe contents of the measuring volume at least at a first probing frequency in correspondence with a first adsorption band of said component, such as N₂O. The spectrophotometer assembly 10 typically includes an IR light source 11, e.g. a broadband source or a scannable source, and a photo detector 12. Light L from the light sources passes through the measuring volume 4, where it can interact with breath including a compound, if present, within the volume before arriving at the photodiode detector. One or more band pass filter 13 can be provided in accordance with target probing frequency range or ranges.

In some embodiments, e.g. as shown, the device comprises one or more of:
- a mouth piece 5
- a particulate filter 6;
- a flow restriction means, e.g. orifice 7;
- a carbon dioxide trap (8);
- a flow meter (9); and
- a pressure sensor (P)

The mouth piece allows a user to blow into the device so as to generate the gas flow that passes the measuring volume 4 and exists at the outlet. For hygienic reasons one or more of the inlet and the mouth piece are preferably embodied as replaceable consumables.

The particulate filter, preferably disposed directly downstream the mouthpiece or the inlet filters out particulates such as droplets from the gas flow as generated by the user. Like the mouth piece and/or inlet the filter is preferably a replaceable consumable, optionally as part of the inlet or mouthpiece.

The flow restriction means 7 restricts a flow towards the probing volume to a predetermined rate, e.g. to 8 L/min. A pressure sensor P measures whether the user supplied flow meets the predetermined rate. When the criterion is met, e.g. by detection of an overpressure by the sensor, the, a trigger can be generated for stating a measurement interval

Flow and measurement duration can determine whether a sufficient volume of gas is sampled. Excess flow of supplied gas can be routed out of the system via an overflow outlet 7a. For reference, a volume of approx. 1.3 L is used in similar handheld devices configured for alcohol detection.

The flow meter 9 can be used to monitor a flow rate of sampled gas. The rate in combination with relevant measurement period can be a measure for the sampled volume.

Trap 8, positioned in the gas flow path upstream the measuring volume filters out carbon dioxide so that it does not obscure presence of N₂O during photo spectrophotometeric detection. The trap is preferably a replaceable trap.

A computing unit 30 can be provided to control one or more of the spectrophotometer assembly, the flow meter, and further electronic components comprised in the device. en meetduur bepalen of voldoende is bemonsterend, ter vergelijking in een alcohol blaastest

FIG 2 illustrates a further embodiment of a breathalyzer device. For reasons of clarity not all components in described in relation to FIG 1 are shown/marked. In an embodiment, e.g. as indicated, the breathalyzer further comprises sorption module 20 that is removably connected downstream the measuring volume. During use the sorption module (e.g. a Tenax sorbent tube having a 5 Angstrom molecular sieve) collects gaseous/volatiles to be detected e.g. N₂O). In another or embodiment, e.g. as indicated, the breathalyzer device is equipped with a selectable bandpass filter 13a disposed in a pathway between a light source and detector of the spectrophotometer assembly. The filter allows detection of a variety of compounds. For example, as best seen in the bottom front-view image of the filter 13a the filter can have a plurality of positions (five indicated), each having a band pass filter allowing passage of light in a band (or ranges) indicative for a specific target compound.

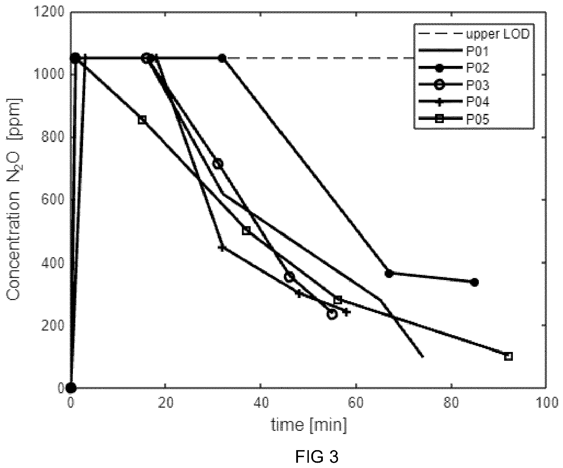

FIG 3 depicts traces of experimentally determined concentration of N₂O in human breath as a function of time (in minutes) after intake of a single dose of laughing gas (at t=0). Traces P01 to P05 related to different individuals. Dashed line LOD indicates an upper limit of detection. The graph confirms that nitrous oxide can be detected in exhaled breath long after an intake.

For the purpose of clarity and a concise description, features are described herein as part of the same or separate embodiments, however, it will be appreciated that the scope of the invention may include embodiments having combinations of all or some of the features described. Of course, it is to be appreciated that any one of the above embodiments or processes may be combined with one or more other embodiments or processes to provide even further improvements in finding and matching designs and advantages. It is appreciated that this disclosure offers particular advantages.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A breathalyzer device (1) for direct and quantitative detection of nitrous oxide (N₂O) in breath as exhaled into the device by a user, the device comprising an inlet (2) for receiving a flow of the exhaled breath, a measuring volume (4) fluidly connected between the inlet and an outlet (3), and an IR spectrophotometer assembly (10) operably configured to probe contents of the measuring volume at a first probing frequency in correspondence with a first adsorption band of N₂O.

2. The breathalyzer (1) according to claim 1, wherein the analyzer is further configured to probe at a second probing frequency overlapping a second adsorption band of N₂O, different from the first.

3. The breathalyzer (1) according to claim 1, according to claim 1 or 2, further comprising a flow meter (5).

4. The breathalyzer (1) according to any of the preceding claims, further comprising an flow restriction orifice configured to restrict an inward flow rate towards the measuring channel (4).

5. The breathalyzer (1) according to any of the preceding claims, further comprising a pressure sensor (6) configured to, upon reaching a pressure threshold, trigger a measurement period for the IR analyzer (10) and the flow meter (5).

6. The breathalyzer (1) according to any of the preceding claims further comprising a selectable bandpass filter disposed in a pathway between a light source and detector of the spectrophotometer assembly (10).

7. The breathalyzer (1) according to any of the preceding claims, configured for detecting presence of N₂O, further comprising an N₂O sorption module (7) that is removably connected downstream the measuring channel (4) for collecting N₂O.

8. The breathalyzer (1) according to any of the preceding claims, further a carbon dioxide trap (8) that is removably provided upstream the measuring channel (4).

9. A method of detecting nitrous oxide (N₂O) in the breath of a user, the method comprising:
providing a breathalyzer, preferably the device according to any of the preceding claims, comprising at least, a measuring volume fluidly connected between an inlet and an outlet of the breathalyzer, and an IR analyzer (10) operably configured to probe contents of the measuring volume
exhaling into the inlet thereby providing a flow of exhaled gas flow through the measuring volume (4), and
obtaining a response of the IR analyzer (10) at a first probing frequency in correspondence with a first adsorption band of N₂O.

10. The method according to claim 9, further comprising
obtaining a response of the IR analyzer (10) at a second probing frequency in correspondence with a second adsorption band of N₂O.
and determining a ratio between the response at the first probing frequency and at the second probing frequency.

11. The method according to any of claims 9-10, further comprising determining a volume of the probed gas.

12. The method according to any of claims 9-11, further comprising, determining whether a volume of breath exhaled into the inlet exceeds a minimum threshold value.

13. The method according to any of claims 9-12, further comprising
passing the exhaled gas downstream the measuring channel (4) over a sorbent configured to sorb nitrous oxide thereby collecting N₂O oxide, followed by
releasing N₂O from the sorbent and determining an amount of released nitrous oxide.

14. Use of analyzing breath as exhaled by a person for a presence of nitrous oxide exhaled to detect whether the person partook in a prior nitrous oxide consumption.

15. Use of the method or the device according to any of claims 1-13, for public safety control, for example traffic safety control.
